# EUROPEAN PATENT SPECIFICATION

(11) **EP 3 577 584 B1**
(45) Date of publication and mention of the grant of the patent: **23.11.2022**
(21) Application number: 17736819.8
(22) Date of filing: 21.06.2017
(51) Int. Cl.: G16H 20/17, G16H 40/67

(54) **COMMUNICATION WITH DRUG DELIVERY DEVICE**
KOMMUNIKATION MIT ARZNEIMITTELABGABEVORRICHTUNG
COMMUNICATION AVEC UN DISPOSITIF D'ADMINISTRATION DE MÉDICAMENT

(30) Priority: 02.02.2017 US 201762453719 P
(43) Date of publication of application: 11.12.2019
(73) Proprietor: West Pharma. Services IL, Ltd., 4366411 Ra'anana (IL)
(72) Inventor: CABIRI, Oz, Hod Hasharon 4526611 (IL); HAMMER, Tal, Ramat-Gan (IL)
(74) Representative: Petraz, Gilberto Luigi
(86) International application number: PCT/US2017/038537
(87) International publication number: WO 2018/144056

(56) References cited:
- WO-A1-2015/104022
- WO-A2-2011/014704
- US-A1- 2013 317 753
- US-A1- 2014 194 817

## Description

### FIELD AND BACKGROUND OF THE INVENTION

The present invention, in some embodiments thereof, relates to an apparatus and a method for communication with a drug delivery device and, more particularly, but not exclusively, to such communication with a server or like computing device, for example via an intermediate device that is likely to be on hand such as a tablet or smart telephone or the like. Tablets and smartphones are becoming ubiquitous and the chances that the patient owns or at least has access to such a device is increasing.

The concept of pairing a remote computing device with an automatic injector is taught in U.S. Pat. Pub. No. 2016/0012205. The concept of remote monitoring of drug delivery is taught in U.S. Pat No. 8,663,201. Furthermore,document WO 2015/104022 discloses methods and systems for establishing wireless communication between at least one portable medical device having a predefined ID and at least one communication unit such as a smartphone for securely pairing the medical device with the smartphone.

In general, however, at the level of the individual patient, such pairings are not used and are not seen to be useful. Patients may not be technically savvy enough or interested enough to make a Bluetooth pairing, particularly for a device that is to be used only once. The device generally performs its function well enough without a remote connection and therefore the patient has little incentive to find and enter a pairing code.

Despite the above, there is a need to track and record user actions and adherence to a medical regime. There is also a need to link the power of general computing devices and networks to medical delivery devices such as auto injectors. Those needs however are not immediately obvious to patients.

### SUMMARY OF THE INVENTION

Injectors are single use devices. Thus a lengthy startup or pairing process or a process requiring any effort on the part of the patient is likely to lead to considerable resistance to use. In particular, the patient should not be required to show any skill, learning, or expertise in order to use the device. The particular end user may indeed use the device only once, or very occasionally. On the contrary, the use of communication should play a part in increasing the usability of the device.

The present embodiments may thus provide automatic pairing based on information that is present and can be sensed automatically when the medical device is activated. The data may include prescription data, phone data, user identification, and/or scanning a barcode on the device packaging.

The communication may enable real time tracking of the medical procedure such as delivery of a substance using an automatic injector, where features measured may include motor load data, back pressure, duration of delivery, non-delivery times and the like. Instructions for use may be transmitted to the user on a remote device based on real time data received from the delivery device.

According to a first aspect of the present invention there is provided a portable medical device as defined in claim 1.

The device may be an automatic injector.

In an embodiment, the electronically readable information is in the form of a barcode or an RFID tag. In an embodiment, the barcode or RFID tag is a part of packaging containing the portable medical device. In an embodiment, the barcode or RFID is attached to the portable medical device.

In an embodiment, the communication unit is configured to carry out the pairing based on receiving pairing information from another device which is based on the electronically readable information. In an embodiment, the electronically readable information includes information identifying the portable medical device, thereby enabling the other device to connect to a server and identify the portable medical device.

The portable medical device comprises a battery isolator that disconnects a battery from the communication unit. The battery isolator is configured to be displaced by the opening of the packaging, thereby activating the portable medical device upon opening the packaging.

The device may provide the other device with a web location to obtain a program to operate with the portable medical device. The device may contain a memory unit with a program that is downloadable to the other device to allow the other device to operate with the portable medical device.

The device may send, via the pairing, dynamic data of a medical procedure being carried out by the portable medical device. The device may send, via the pairing, current state data of the portable medical device.

In an embodiment, the current state data comprises one member of the group consisting of: temperature and readiness.

The device may send operational data at predetermined intervals as accumulated since a respective preceding interval, or send all measurements as accumulated from a start of operation at each predetermined interval.

The device may be paired with a communication device comprising: a communication unit; a recognition unit for recognizing identification information passively readable or held externally on the portable medical device or on packaging containing the device; a pairing unit connected to the recognition unit to pair the communication device with the portable medical device when recognized, thereby to open a first channel of communication with the portable medical device; wherein the communication unit is configured to open up a second channel of communication with a server remotely over a network, thereby forming a communication route between the portable medical device and the server via the first and second communication channels. Such a device may, for example, be a mobile phone or a smartphone.

According to an embodiment of the present invention there is provided a system for carrying out a medical procedure, the system comprising the portable medical device as described above, a remote server and another device, wherein the another device comprises an apparatus for connecting the portable medical device and a server, comprising: a communication unit; a recognition unit for recognizing identification information passively readable or held externally on the portable medical device or on the packaging containing the device; a pairing unit connected to the recognition unit to pair the apparatus with the portable medical device when recognized via automatic displacement of the battery isolator from the portable medical device upon opening of the packaging containing the portable medical device, thereby opening a first channel of communication with the portable medical device; wherein the communication unit is configured to open up a second channel of communication with the server remotely over a network, thereby forming a communication route between the portable medical device and the server via the first and second communication channels.

In an embodiment, the recognition unit is configured to obtain data from the portable medical device. In an embodiment, the recognition unit comprises an RFID scanner or a barcode scanner. In an embodiment, the data obtained from the portable medical device and sent to the server is sufficient to allow the server to identify the portable medical device.

The device may obtain, via the first channel, dynamic data of medical process carried out by the portable medical device, and may pass on the dynamic data via the second channel to the server.

The device may receive data from the server via the second channel and may display the data to a user.

The device may obtain an identification of the portable medical device via the recognition unit, and may use the obtained identification to provide an identification prefix for communication data packets.

In an embodiment, the communication data packets with the identification prefix are for the second communication channel. In an embodiment, the communication data packets with the identification prefix are for the first communication channel.

In an embodiment, the identification prefix comprises data contained on the portable medical device. In an embodiment, the identification prefix comprises a derivation of data contained on the portable medical device. In an embodiment, the derivation comprises one-way hashing.

An embodiment may accumulate operational data from the portable medical device for sending to the server at predetermined intervals, the operational data accumulation beginning at a start of operating the portable medical device.

An embodiment may send operational data, as accumulated from the start, at each of the predetermined intervals, or send at each interval, measurements accumulated since a preceding interval.

An embodiment may receive a temperature measurement from the portable medical device and calculate a wait time for operating the portable medical device based on the temperature measurement.

An embodiment may receive from the portable medical device any of an elapsed time, a load on a controlling motor, a substance volume, a pressure, a temperature, and a number of revolutions of the controlling motor and display at least one member of the group consisting of the elapsed time or a remaining time or a quantity of substance delivered or a quantity of substance remaining.

An embodiment may determine from any of an elapsed time, a load on a controlling motor, a substance volume, a pressure, a temperature, and a number of revolutions of the controlling motor, that a procedure or dosage is incomplete and indicate the incompleteness.

An embodiment may determine that a procedure or dosage is complete and display steps to be carried out following completion of the procedure or dosage.

An embodiment may send the identification information to the server to find further information at the server regarding a procedure or dosage indicated along with the portable medical device, the apparatus being further configured to retrieve and display the further information.

An embodiment may connect to the server to obtain a schedule for use of one or more of the portable medical devices and to provide schedule-related information including timed reminders.

According to a second aspect of the present invention there is provided a method of monitoring usage of a portable medical device as defined in claim 35.

In an embodiment, the method comprises providing each of a plurality of users, the plurality of users being volunteers in a clinical trial relating to the portable medical device, with the same system and obtaining data regarding usage of the portable medical device by respective volunteers and sending the data to the server.

In an embodiment, the recognition unit is configured to obtain data from the portable medical device, the data comprising date information, thereby enabling the system to warn a user if the portable medical device is out of date.

In an embodiment, the method comprises pairing a portable medical device and connecting with a server via a mobile telephony device, comprising: reading identification information provided on the portable medical device or on packaging associated with the portable medical device, the identification information being provided for reading while the portable medical device is passive; operating the portable medical device and carrying out pairing with the portable medical device based on the identification information; connecting with the server based on the identification information; and providing guidance screens on the mobile telephony device based on data from the server and the portable medical device to guide a user to use the portable medical device.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. The computers discussed herein or otherwise applicable to the present embodiments may be networked and may be a server, a desktop computer, a laptop, a tablet, a pod, a mobile telephone including a smartphone, or any other computing device, and connections may be based on the Internet protocol or may use Bluetooth^{™} or may involve hotspots or wifi or cellular communications or other radio communications or wire communications or infra-red. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1 is a simplified exploded diagram of an injector device that may be used with the present embodiments;
Fig. 2 is a view of the injector device of Fig. 1 in an assembled state, with the outer housing made transparent to show internal detail;
Fig. 3 is a conceptual block diagram of a first embodiment of the present invention;
Fig. 4 is a simplified view of an initial screen of a patient's computing device upon launching the application and prior to pairing with the injector device, according to an embodiment of the present invention;
Fig. 5 is a simplified view of a screen of a patient's computing device following launching and explaining how to scan the barcode so as to carry out pairing, according to an embodiment of the present invention;
Fig. 6 is a simplified view of a screen shown after successful scanning and instructing the user to open the packaging;
Fig. 7 is a simplified view of a screen of a patient's computing device following opening and removal of the battery isolator, wherein the device warms up for activation, according to an embodiment of the present invention;
Fig. 8 is a simplified view of a screen of a patient's computing device following warming up and instructing the user to activate the device for injection and ready for delivery;
Fig. 9 is a simplified diagram illustrating opening of the packaging according to an embodiment of the present invention;
Fig. 10 is a simplified view of a screen of a patient's computing device during the course of the drug delivery procedure, according to an embodiment of the present invention;
Fig. 11 is a simplified view of a screen of a patient's computing device following successful completion of the procedure, according to an embodiment of the present invention;
Fig. 12 is a simplified view of a screen of a patient's computing device showing scheduling of a course of treatment, according to an embodiment of the present invention;
Fig. 13 is a simplified view of a screen of a patient's computing device in the event that delivery was not completed successfully, according to an embodiment of the present invention; and
Fig. 14 is a simplified view of a test screen set up to monitor back pressure and temperature in the delivery device according to an embodiment of the present invention.

### DESCRIPTION OF SPECIFIC EMBODIMENTS OF THE INVENTION

Certain terminology is used in the following description for convenience only and is not limiting. The words "lower," "bottom," "upper" and "top" designate directions in the drawings to which reference is made. The words "inwardly," "outwardly," "upwardly" and "downwardly" refer to directions toward and away from, respectively, the geometric center of the apparatus, and designated parts thereof, in accordance with the present disclosure. Unless specifically set forth herein, the terms "a," "an" and "the" are not limited to one element, but instead should be read as meaning "at least one." The terminology includes the words noted above, derivatives thereof and words of similar import.

It should also be understood that the terms "about," "approximately," "generally," "substantially" and like terms, used herein when referring to a dimension or characteristic of a component of the invention, indicate that the described dimension/characteristic is not a strict boundary or parameter and does not exclude minor variations therefrom that are functionally similar. At a minimum, such references that include a numerical parameter would include variations that, using mathematical and industrial principles accepted in the art (e.g., rounding, measurement or other systematic errors, manufacturing tolerances, etc.), would not vary the least significant digit.

The present invention, in some embodiments thereof, relates to apparatus and a method for communication with a medical device such as an automatic drug delivery device and, more particularly, but not exclusively, to establishing communication between the medical device and a central server, via an intermediate computing device that is likely to be in the possession of the patient, such as a tablet or smart telephone or the like. Communication may be established and maintained for the duration of a medical procedure such as injection of a drug.

Embodiments may provide automatic pairing between a user's computing device and the drug deliverer or other medical device. The pairing does not require user entry of data but rather may be based on prescription data, phone data, user identification and/or scanning a barcode on the device packaging or the device itself. In particular the data may be data which is passive and can be scanned, so that the data can be read before the device is switched on.

The embodiments may obtain measurements from the medical device to track the procedure, such as duration or elapsed time of delivery of the dose, motor load data from the motor of an automatic injection device to track injection progress, back pressure, number of revolutions of the motor, rate of revolutions of the motor, etc. and may obtain determinations as to whether the dose has been completed or has been interrupted before completion. Instructions for use may be transmitted to the user based on real time data received from the delivery device, so that the user can be told the next steps in the procedure or can be given alternative instructions in the face of a malfunction etc.

A link between a central server and a drug delivery device via a user's computing device, may serve certain functions:
1) Tracking adherence to dosing regimens and user instructions both in the cases of clinical trials, and in normal patient care;
2) Identification of points of failure;
3) Giving up to date and detailed information to medical personnel on medical conditions and treatment;
4) Giving real time feedback to a user during operation for example:
   a. Instructions
   b. Progress information
   c. Encouragement
   d. Warnings if anything goes wrong, and what do in such cases if detected
   e. Commercial information
5) Identifying the patient and preventing or identifying misuse of drugs.
6) Supplying data for improvement of the device.

As discussed in the background, not all of the above functions are of obvious significance to the patient, who has to carry out a Bluetooth pairing and find a pairing code every single time. In fact since the device works without the pairing, and some of the points are of marginal benefit to the patient, others not aimed at the patient at all, compliance is generally very low if state of the art pairing is required.

An embodiment of the present invention makes compliance more likely by removing the need for the patient to enter any data in order to carry out Bluetooth pairing. Instead a barcode or RFID of the injector or other portable medical device is scanned and used to automatically carry out pairing. The barcode or RFID is typically contained on the packaging but may alternatively be located on the medical device itself. The paired user device then automatically seeks out the relevant server and thus makes a three-way connection between the injector, the user device, and the server based solely on the scan.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

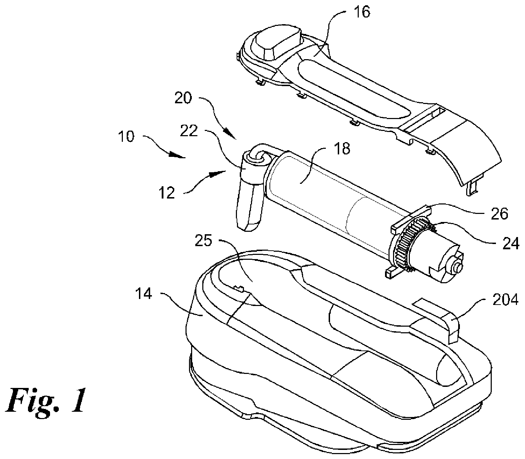

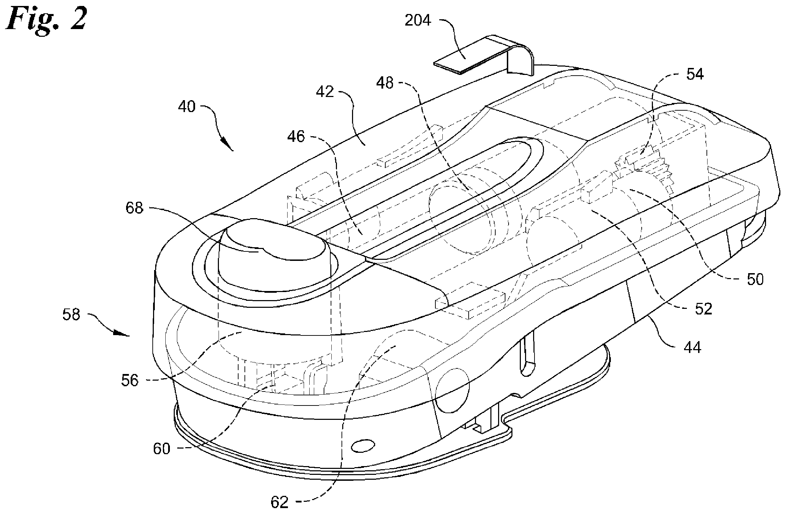

Reference is now made to the drawings in which Figures 1 and 2 illustrate one example of a portable medical device, in particular an injection device, which is suitable for use in the present embodiments. Figure 1 illustrates a device 10 for drug injection. Device 10 includes a cartridge 12, a housing 14 that covers the device from the outside, and a removable cover 16 which fits onto the housing 14. The cover 16 may be removed to give access to the cartridge, for either insertion or removal.

The cartridge 12 has a barrel reservoir 18 for holding a fluid to be injected and a fluid path 20 for carrying the fluid from the reservoir 18 to the point of injection. The fluid path 20 comprises a needle, not shown in this figure, under a needle cover 22. The fluid path 20 is kept sterile by being sealed from the device 10, and the only contacts with the device 10 are mechanical contacts which act through the seal without compromising the integrity of the seal and thus of the sterility. Thus the device 10 as a whole need not be sterile. As a result the cartridge 12 can be inserted and removed without compromising its sterility.

Cog 24 is engaged by a mechanism, typically an electric motor to push a plunger within the barrel 18 to carry out the injection. Engagement frame 26 on the cartridge engages a holding recess 25 within the device 10 to position the cartridge 12 correctly .

In some of the present embodiments, the number of rotations of the cog 24 are tracked so that the position of the plunger within the cartridge 12 is known, and thus the extent of advance and the amount of remaining fluid may be obtained. Additionally or alternatively, rate of rotation of the cog 24 may be measured to provide an indication of how the injection is proceeding

In some of the present embodiments, the back pressure on the plunger may be manifest as resistance by the cog 24 to the turning of the motor, and may be measured. Thus the pressure within the barrel 18 may be obtained. For example high pressure or high resistance may indicate a blockage and a low pressure or resistance may indicate that the needle is not in position and the injection is being made into the open air.

Reference is now made to Fig. 2 which is a simplified diagram showing a version 40 of the device of Fig. 1 in which a drive chain extends the needle to the injection point. That is to say, the device is placed on the user's skin with the needle retracted and a drive chain is activated to push the needle downwards under the skin of the user. The upper part of the housing and of the cartridge are made transparent to show the parts of the drive chain.

The drive chain is mechanically connected to the fluid path to operate the needle. The mechanical connection simply pushes on the fluid path from the outside and does not compromise the sterility within the fluid path. The mechanical connection thus operates the needle while maintaining the fluid path sterility.

One non-limiting embodiment of the drive chain is now described with reference to Fig. 2. The embodiment of Fig. 2 is based on a wound spring, although embodiments based on other sources of tension, on electric motors, on pressing by the user, and on other mechanisms may also be used. The drug delivery device 40 includes upper and lower longitudinal surfaces 42 and 44, wherein lower longitudinal surface 44 is intended to be placed against the skin. Reservoir 46 contains a drug to be injected and plunger 48 empties the drug into the needle for injection. The plunger is operated by a plunger operation mechanism including a small electric motor 50 and battery 52, and a motion translation arrangement 54. A needle insertion mechanism 56 is located at a forward end 58 of the drug delivery device 40 to insert needle 60 into the skin of a patient and withdraw the needle afterwards.

A motion source may be provided to operate the needle 60. The motion source may in one embodiment be a tensioned wheel 62. In other embodiments a plunger may be pushed by a spring.

A user-operated control for the motion source may comprise button 68. It is noted that the button 68 may be a separate component that is compressible or it may be a flexible part of the housing that can be pressed. As the button 68 is pressed once, the wheel 62 rotates half a turn. The motion of the half turn is translated into an insertion motion for needle 60, thus inserting the needle 60. In an embodiment, the button 68 is a separate component from the device housing, so that the housing shields the button 68 from accidental pressing. To reach the button 68, the cover part of the housing may be lowered, thus providing a two stage activation system and adding safety to the device 40.

A second wheel may be provided on the other side of the button 68, not visible in the present figure, to provide more balanced motion.

In one embodiment, as the wheel 62 is released a second time, it rotates on the second part of a complete rotation, and the motion is translated into retraction of the needle 60.

In an alternative embodiment, the needle may not be retracted, but rather the base may extend to shield the needle when the device is removed from the skin.

Alternatively or additionally, the linear movement of the needle retraction may for example be by means of multiple actuators or springs moving the needle in opposite directions. In some embodiments, the activation button may return to the original position and/or be locked after injection.

Alternatively or additionally, needle retraction may be activated by a switch. For example there may be a needle retraction switch and/or after needle insertion the activation button may be reused as a needle retraction switch.

In embodiments, the current position of the needle in the extension - retraction cycle may be sensed.

Thus the device has a housing, and button 68 which operates the drive chain as will be described in greater detail below. The housing is shaped and sized to be held in a one handed grip, in the same way that a computer mouse is so designed. The button 68 is positioned with the one-handed grip in mind to be operated by the fingers while in the one-handed grip.

The device may have an adhesive layer at the base for attachment to the skin of the patient so as to hold the device still for the duration of the injection. For certain injections there may be a need to inject slowly so the device needs to be in the same position for several minutes. Preferably, the device is attachable and detachable via the adhesive layer using the one-handed grip and a rolling motion between the front and back of the device. Thus the device is firmly positioned and adhered and then peeled off after use.

As mentioned, the cover 16 may be removable to allow loading and removal of the reservoir via the opening made in the housing.

The entire cartridge is removed, thus taking out the reservoir with the accompanying fluid path.

The above description is simply one example of a number of injector devices to which the present embodiments are applicable, but shows how various operations of the injector can be measured and, so as to make the measurements available remotely.

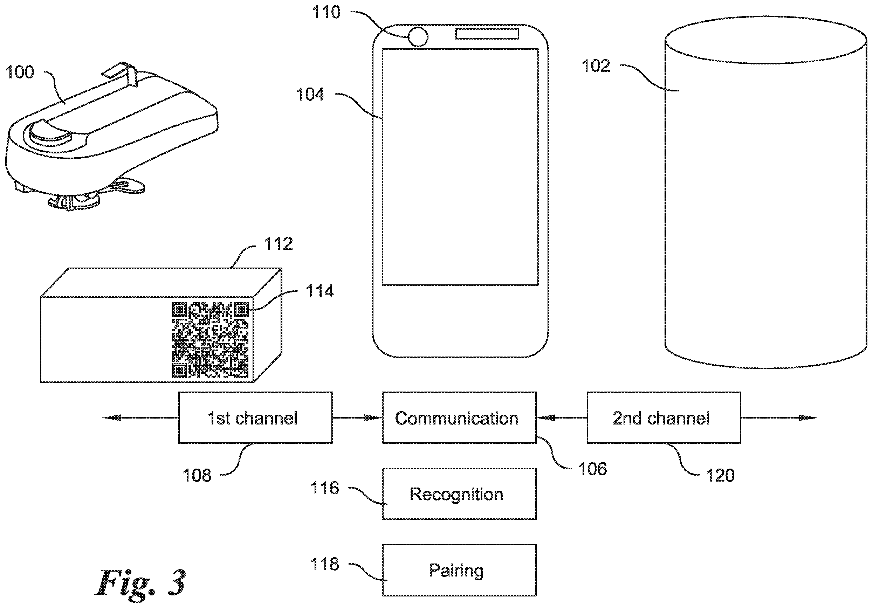

Reference is now made to Fig. 3, which is a simplified diagram showing a system that includes a portable medical device 100, here shown by way of example as the injector device of Figs 1 and 2 but this is only by way of example and any portable device for carrying out medical procedures, particularly those which a patient may carry out at home, are contemplated. The system further includes a remote server 102 and a communication-enabled computing device 104. The computing enabled communication device 104 may be any device available to the patient which is able to run autonomous applications or apps, and has communication abilities, for example a mobile telephone or a pad or pod device. The computing device 104 may serve to connect the portable medical device 100 with the remote server 102 and during the connection may provide useful information to the patient. It is not required in the present embodiments that the computing device 104 exerts any control over the medical device 100. On the contrary, in some embodiments it is a feature that the portable medical device is not controlled via the connection but only by physical action of the patient. One reason for such a feature is so that the operation of the device cannot be hacked.

The portable medical device may comprise a unit for carrying out a medical procedure, for example the injector as described above. A monitoring unit may include measuring devices or sensors which monitor states or operation of the device in connection with the medical procedure. The device further includes a communication unit which is designed to pair with another device to send data obtained from the monitoring unit. The portable medical device is packaged with passive electronically readable information which enables the other device to carry out pairing, so that a connection can be made easily and simply without requiring an end user to type in pairing codes or the like.

The electronically readable information may be in the form of a barcode or an RFID tag, and the barcode or RFID tag may be built into the device, attached, say as a sticker to the wall of the device or may be a part of packaging containing the portable medical device.

The barcode may be placed at a location which allows for external scanning when the packaging is still on the device. Thus it may be on the outside of the packaging, or it may be on the inside of the packaging opposite a transparent part of the packaging, or it may be on the device itself, opposite a transparent part of the packaging. Such considerations need not apply to an RFID tag.

The portable medical device may carry out pairing based on a code that the other device obtains from the electronically readable information, so that the other device may for example scan the barcode on the medical device, obtain the pairing code and carry out pairing.

The electronically readable information may additionally include information identifying the portable medical device. The connecting device may use that information to connect to a server over a second connection and identify the specific portable medical device.

The portable medical device includes a battery isolator that disconnects a battery from the communication unit. The battery isolator is designed so that it is displaced upon opening of the packaging. Thus opening the packaging automatically activates the portable medical device.

In general the connection device already has a program or "app." for operating the portable medical device. However, in case such a program is not present on the connecting device, the electronically readable information may further provide a web location to obtain the necessary program.

In one embodiment, the portable medical device may contain a memory unit with a program that is downloadable to the other device directly so that internet downloading is not required.

The portable medical device may send, via the pairing link, dynamic data of a medical procedure being carried out, such as an injection operation. The dynamic data may include current state data such as temperature and readiness.

The data may be sent when obtained, or may be accumulated into a packet which is sent at regular intervals. In an embodiment, all the data accumulated since the start of operation is sent each time a packet is sent. In this way, missing or dropped packets do not lead to loss of information.

The other device, the connecting computing device, may include a communication unit 106, which may manage a first communication channel 108 between the computing device 104 and the medical device 100. The first communication channel 108 is typically a short range link and may be a short range radio link, say based on Bluetooth^{™} or may be an infra-red link or the like.

In general, devices according to the Bluetooth standard are required to pair. One of the devices discovers the other device and then the discovery is confirmed via user input, first to recognize the discovered device and then secondly to input a pairing code. The user input may ensure that unintended or unwanted connections are not made. Pairing that requires user input is usually one time and once a device is discovered by another device and confirmed then future connections are generally automatic.

However medical devices such as injectors are generally single use devices. Thus pairing such devices requires user input every single time, and patients, often elderly or ill or in a hurry or some combination thereof, may simply leave out the pairing procedure, either because they do not consider it necessary or because they do not know how to perform pairing or for any other reason.

The computing device 104 however is generally equipped with a camera 110, and the camera 110 is able to scan the medical device 100 or the packaging 112 provided with the medical device and serve as a recognition unit. Barcode 114 on the device 100 or packaging 112 - here shown on the packaging 112 by way of example, may provide identification information, allowing pairing to proceed automatically. That is to say the patient simply points the camera 110 at the barcode 114 and the computing device 104 automatically recognizes the barcode using recognition unit 116 and pairs with the medical device 100 without requiring further patient input, thus activating the first communication channel 108. The recognition unit 116 need not be associated with a camera but may for example be associated with a radio frequency identification (RFID) scanner, in which case the identification information may be provided on the medical device or packaging as an RFID tag. The recognition unit 116 carries out recognition of the output of a sensor that can sense identification information on the portable medical device 100.

Pairing unit 118 may use the data from the recognition unit to carry out pairing and recognize the medical device 100 as an authorized Bluetooth^{™} pairing. Once the medical device 100 is recognized and paired with, then a second channel of communication 120 is opened with server 102 remotely over a network. The second communication channel 120 may for example use a cellular connection or locally available Wi-Fi, or an Internet connection and may use GPRS over GSM or CDMA or may directly use the IP protocol or some combination. Overall there is formed a communication route between the portable medical device 100 and the server 102 via the first and second communication channels 108 and 120 respectively.

The barcode 114 or RFID or the like may provide information to identify the specific medical device. In addition they may provide enough information to identify the associated patient or the associated prescription. Alternatively the computing device 104 may identify the patient or the associated prescription, or in some embodiments, the patient may have been provided with the prescription in electronic form. In all of the above cases and in others, instructions relating to the specific patient or specific prescription may be available on computing device 104 and may be shown to the patient at the time at which the device is used.

The computing device 104 may obtain, through the first channel 108, dynamic data of the process carried out by the portable medical device 100. For example, the channel 108 may provide any measurements made on the medical device, or may provide update information regarding the process being carried out. Computing device 104 may then relay the dynamic data via second channel 120 to server 102.

The server may then make use of the measurements or other dynamic data and display relevant information to the user on the computing device 104. Thus for example the medical device 100 may measure temperature, and the server 102 may determine that at a certain temperature the device 100 is safe to use and may then display on the computing device 104 the information that it is now safe to go ahead and operate the medical device 100.

In an example, the portable medical device 100 is an automatic injector device as shown in Fig. 3.

As discussed, barcode 114 or other machine readable information on the medical device 100 or packaging 112 may identify the specific medical device. The device identification may be used directly in packet headers as a packet identifier or identifier prefix or identifier suffix, to provide easily identifiable communications that cannot be hacked by anyone without physical access to the device. In another embodiment the identification in the packet header may be derived from the identification information, say using a one-way hash, or seeding a pseudorandom generator, so that physical access to the medical device would not be sufficient to hack the communications.

The identification in the packet may be used for the first communication channel 108 or the second communication channel 120 or both communication links.

The data packets may carry all kinds of information from the medical device, and there is a risk of some packets getting lost or dropped over the network. Therefore, in an embodiment, each packet carries not only all of the latest measurements but also all of the data accumulated since operation of the current medical device began. Thus if a packet is dropped there is no information loss. That is to say, either the medical device or the computing device, may accumulate operational data since the start of the current operation of the portable medical device and all of the accumulated information is added to each successive communication. Communications may be sent at successive intervals and the intervals may be fixed or varying intervals, and the accumulated operational data since the start of the current operation of the portable medical device may often fit easily into a single packet. If not then some of the communications, particularly the later communications, may consist of two or more packets.

The information accumulated may include measurements, as well as data of other kinds.

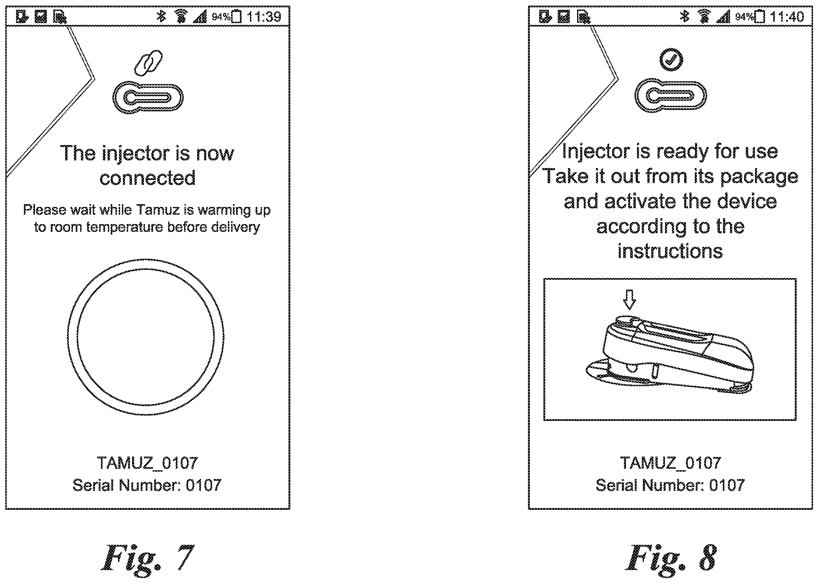

One of the kinds of data that may be measured and transmitted is temperature. Often, materials for injection need to be refrigerated or frozen and cannot be used until they reach a certain temperature. Thus the current temperature of the material inside the device may be transmitted, and the computing device may indicate that the material has reached a temperature which makes the device possible to use. Alternatively the temperature measurement may be entered into a calculation to indicate a safe waiting time before the medical device may be used. The waiting time may be a function of the internal temperature of the material in the device and the ambient temperature, as discussed below in respect of Fig. 7.

Data that may be measured or obtained on the medical device and transmitted may include an elapsed time, say the amount of time that the injector has been operating or that an injection has been proceeding. The load on the controlling motor may be measured and transmitted. A high load may indicate a blockage and may trigger information to be displayed to the patient. A low load may indicate that the needle is not in position and may likewise trigger display of relevant information. A volume of the material or substance that has been delivered or injected may be measured, say based on the current position of the plunger in the cartridge. Pressure may be measured in the device to provide information about blockages or about the needle not being in the correct position. Temperature has been discussed above and may be used to indicate whether the substance has reached a usable temperature following refrigeration. In addition, the temperature of the device may have been recorded since the device was filled and may be used to determine whether the device has been stored properly and whether the substance is still safe to use or may have become denatured or infected. The number of revolutions of the controlling motor or cog wheel or the number of revolutions per unit time may be used to indicate progress or rate of progress.

The screen on the computing device may show to the user the elapsed time or the remaining time or the quantity of substance delivered or the quantity of substance remaining, or any safety directions that may arise from anomalies being measured.

The measurements may be used to determine that the dose is incomplete, and the information about the incomplete dose may be sent to the prescribing doctor or to the patient. The injection or other process or procedure may be part of a clinical trial so that data about an incomplete dose may be important for the conduct of the trial.

Likewise the measurements may determine that the procedure or dosage is complete and may indicate on the computing device that the procedure is completed or may display steps to be carried out following completion, such as how to safely remove the medical device or what to do next, or what indications of possible side-effects to look for.

For example, a prescription may be sent to a user's smartphone by the doctor or clinic, or may be sent in response to connecting the medical device, based on barcode information which points to the relevant data on the server. Thus, the phone may schedule the treatment or treatments and remind the user and/or recognize the proper medication and time. Data and/or information may be supplied to and/or from other related sources. For example, a doctor or health center may be informed of the user's schedule and/or actions. For example, data and/or incentives messages may be sent to the user from the health center coordinated with the dosage schedule and/or delivery device status. Thus either the user's computing device or the server may provide a use schedule for the user. The prescription may relate to a course of treatments over a period of time, and the transmissions may provide the user with a schedule for the treatment, which may include timely reminders. Likewise the server may record patient compliance with the schedule.

The identification information may lead to further information at the server 102 regarding the procedure or dosage which can be displayed to the user.

It is noted that wherever the term "displayed" is used, the same information may alternatively or additionally be provided in voice form.

In greater detail, the entire procedure from removing the injector from the packaging to completion of the injection or other procedure is designed for integration between the delivery device, the patient's computing device and the remote server.

The computing device may present user and drug information, including for example the user name, the drug name, whether the drug fits a prescription and whether it is the proper time for delivery. The device may give instructions based on the data, for example to open the packaging and start delivery and/or to return the medicine because it is wrong or outdated and/or to put the medicine back in the refrigerator because it is not yet time for delivery.

In some embodiments, functioning of the delivery device may be tied to the computing device, for example the delivery device may be disabled until a code is received from the computing device that the time, user and/or drug are correct and/or the proper warm up time has been completed - see Fig. 7. In this way misuse and/or overdose may be avoided.

Optionally, the device may require the user to identify himself, for example using a password and/or bio-identification (e.g. a fingerprint reader) before allowing him/her to continue.

The packaging may include a sensor and/or switch. For example the packaging may send a message to the computing device if it is not properly sealed, for example if it was unsealed prematurely and/or has been improperly stored, for example the packaging may include an indicator of exposure to high temperatures. Optionally, the packaging may switch on the delivery device and/or initiate a process in the computing device when the packaging is opened.

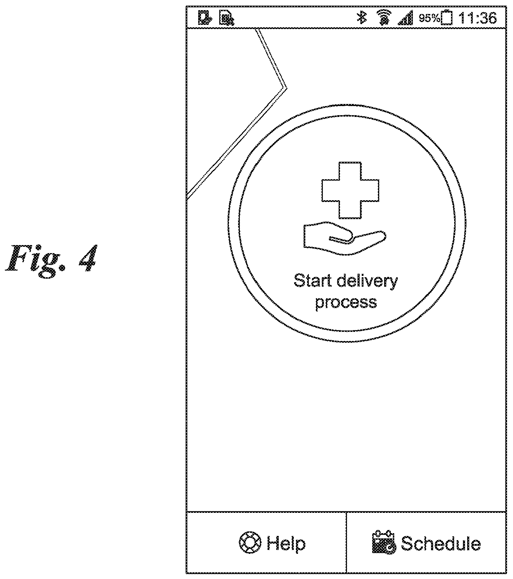

Reference is now made to Fig. 4, which illustrates an initial screen, or home screen, shown when the application is launched on the mobile phone. The idea is to launch the application first, before the injector itself has been opened and activated and to carry out the activation stages under instruction from the mobile phone application.

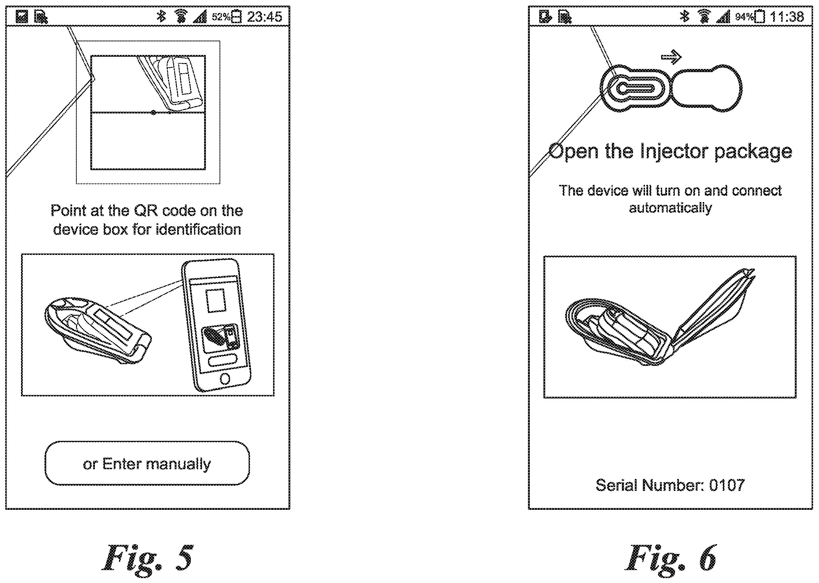

Reference is now made to Fig. 5, which illustrates an exemplary screen shown on the patient's computing device after the initial screen of Fig. 4. At the beginning of the process the user may be invited to scan a barcode or QR code on the drug and/or device packaging and the screen shows the instruction to scan as well as an example showing what the barcode looks like.

Reference is now made to Fig. 6, which is an exemplary screen shown after scanning, and which instructs the user to open the packaging.

Reference is now made to Fig. 7, which shows the state of the telephone after the battery isolator has been removed, following opening of the packaging. The device is activated by removal of the battery isolator and pairing is carried out. Following pairing the application receives the temperature measurement or readiness status from the device and displays the status to the user.

In some embodiments when the packaging is opened the phone already has all of the necessary pairing information from the prescription, the barcode, and/or an external data source associated therewith (for example over the Internet). The delivery device and computing device may automatically and safely pair without requiring involvement of the user, as explained above.

In some embodiments there may be a time out in the process. In some embodiments, a time out may be called for checking data and/or getting authorization and/or for a physical check, for example requiring a patient to perform a blood sugar test and/or for a drug to warm up. Optionally, a time out may be purely time dependent for example the drug may be given a fixed time to reach room temperature, sensor mediated, for example a sensor may measure drug temperature and a time out may last until the drug reaches a proper temperature, and/or a combination thereof, so that the time out may be adjusted based on an external temperature for example measured by the cell phone or an external sensor.

The patient's computing device may give instructions and/or information to the user coordinated to data received. For example, data may be received from the delivery device and/or over a network. The computing device optionally tracks and/or displays progress during delivery.

It is noted that there are separate stages of firstly opening the packaging and removing the isolator to connection of the battery, secondly waiting for the device to warm-up or otherwise initialize, and thirdly removing the device from the packaging and placing on the skin at the injection site.

Reference is now made to Fig. 8, which illustrates the screen on the user's computing device after warming up or otherwise initializing. The injector is ready for drug delivery, and the user is instructed to remove the device from the packaging, place the device on the injection site and activate the device for injection. The screen may include instructions for placing and attaching the device on the skin and pressing the activation button.

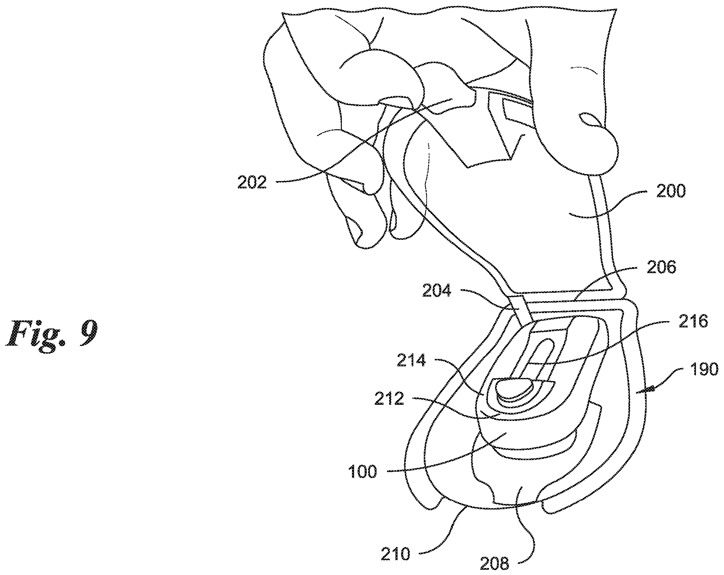

Reference is now made to Fig. 9, which illustrates the medical device 100 in the packaging 190 as the packaging 190 is opened. The patient opens packaging 190 to obtain portable medical device 100, in this case an automatic injector, by pulling up cover 200 using pull tab 202. A battery isolator 204 is removed when the cover 200 is opened, thus activating the device 100. The cover 200 may open by rotating at hinge 206 against base 208 of the packaging 190. The packaging may have a rim 210.

The medical device 100 is designed for single handed operation and thus comprises a wide gripping area 212 in front, a thumb indentation 214 and a narrow gripping area 216 at the rear side.

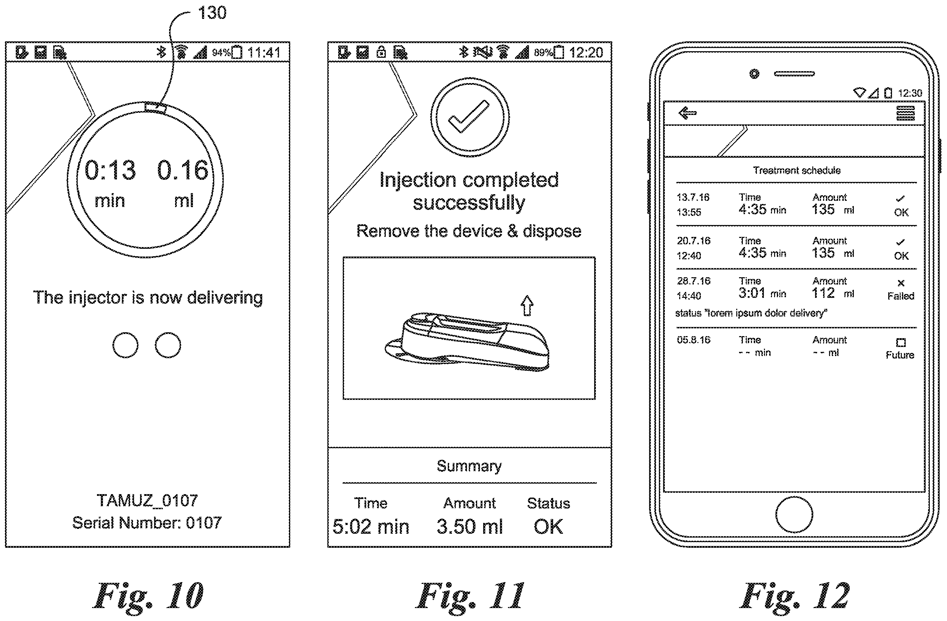

Figure 10 illustrates the screen as the injection proceeds, showing both the elapsed time and the number of milliliters delivered. As the delivery progresses, the circle 130 is filled out and the elapsed time and amount delivered incrementally.

Fig. 11 illustrates the screen after injection is successfully completed.

A summary of the delivery may include elapsed time, size of dose delivered and status, successfully completed, partially completed etc. The summary may be delivered to the user and/or to a medical aid and/or doctor and/or sent to a database over a network. Follow-up medical instructions may include instructions for acquiring, storing and/or taking the next dose and/or follow up medical visits and/or follow up procedures and/or warnings about symptoms of dangerous post-treatment conditions. Follow-up messages may be timed. For example, a warning message may be sent an hour after the injection to check for signs of a reaction.

Reference is now made to Fig. 12, which illustrates a treatment schedule for a course of injections. In the left hand column is shown the date and time of the scheduled treatment. In the center columns are shown the elapsed time and doses actually delivered. In the rightmost column are shown the status, OK or successfully completed, failed, for those operations which do not complete successfully, and future, for procedures whose time has not arrived.

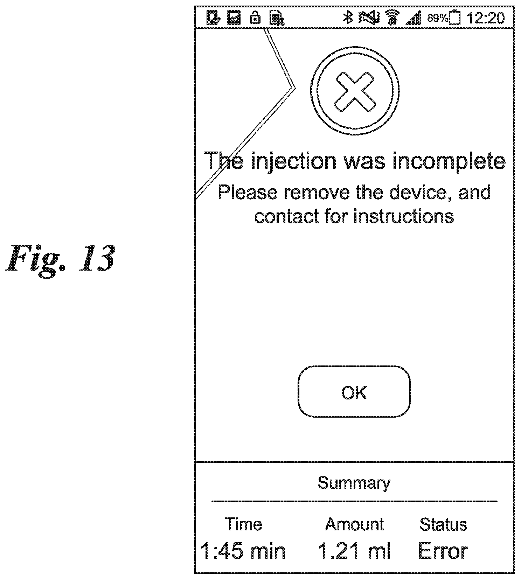

Fig. 13 illustrates an exemplary screen that may be displayed if the delivery is not completed successfully.

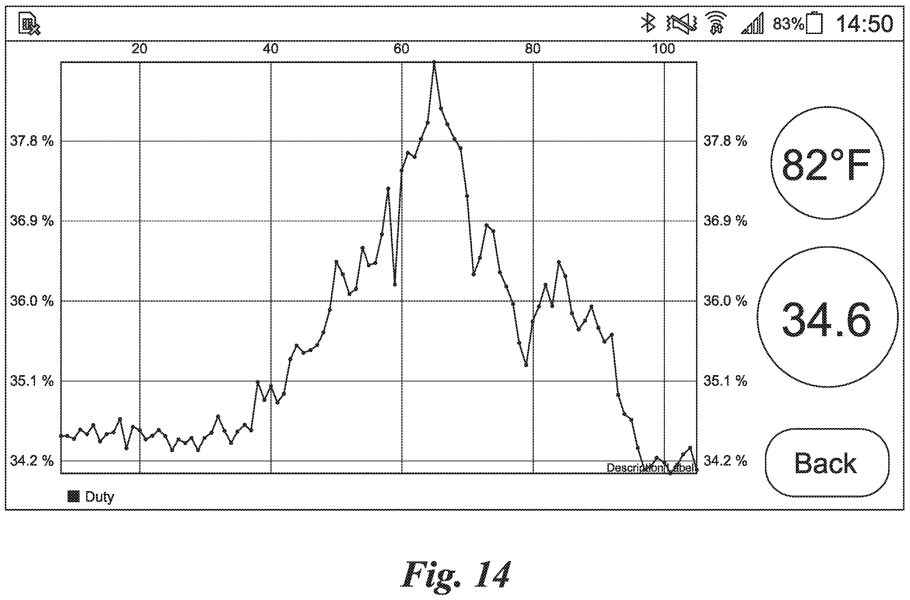

Reference is now made to Fig. 14, which illustrates an exemplary screen that may be used for clinical trials of the device, as opposed to use with patients. The screen presents the on-line motor load graph. The graph shows the on-line developing system load, which allows the back-pressure values to be calculated. The device measures the system/motor load values, which allows back pressure to be calculated, so that the screen may graph the back pressure as a percentage of maximum load against delivery time and also as a percentage of the total delivery time. The screen also includes temperature measurements in centigrade and Fahrenheit. The back pressure is related to other parameters so that monitoring of the back pressure allows for study of the operation and improvement of the device.

It is expected that during the life of a patent maturing from this application many relevant mobile medical devices and automatic injection devices will be developed and the scope of the corresponding terms are intended to include all such new technologies *a priori.*

The terms "comprises", "comprising", "includes", "including", "having" and their conjugates mean "including but not limited to".

As used herein, the singular form "a", "an" and "the" include plural references unless the context clearly dictates otherwise.

It is appreciated that certain features of the invention, which are, for clarity, described in the context of separate embodiments, may also be provided in combination in a single embodiment and the present description is to be construed as if such a combination is explicitly set forth herein. Conversely, various features of the invention, which are, for brevity, described in the context of a single embodiment, may also be provided separately or in any suitable subcombination or as suitable in any other described embodiment of the invention, and the present description is to be construed as if all such combinations and subcombinations are explicitly set forth herein. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art.

Citation or identification of any reference in this application shall not be construed as an admission that such reference is available as prior art to the present invention. To the extent that section headings are used, they should not be construed as necessarily limiting.

## Claims

1. A portable medical device (100) comprising a unit to carry out a medical procedure, a monitoring unit to monitor said medical procedure and a communication unit configured to pair with another device to send data of said monitoring unit, the portable medical device being packaged with passive electronically readable information to enable said another device to carry out said pairing, the portable medical device further including a battery isolator (204) that disconnects a battery from the communication unit, the battery isolator (204) being configured to be displaced upon opening of a packaging containing the portable medical device, thereby activating the portable medical device upon opening of the packaging.

2. The portable medical device (100) of claim 1, wherein the medical device is an automatic injector.

3. The portable medical device (100) of claims 1 or 2, wherein said electronically readable information is a barcode (114), QR code, or an RFID tag.

4. The portable medical device (100) of claim 3, wherein the barcode (114), QR code, or RFID tag is a part of the packaging containing the portable medical device.

5. The portable medical device (100) of claim 3, wherein the barcode (114), QR code, or RFID is attached to the portable medical device.

6. The portable medical device (100) of any one of the preceding claims, wherein said communication unit is configured to carry out said pairing based on receiving pairing information from said another device, which is based on said electronically readable information.

7. The portable medical device (100) of any one of the preceding claims, wherein said electronically readable information includes information identifying said portable medical device, thereby enabling said other device to connect to a server and identify said portable medical device.

8. The portable medical device (100) of any one of the preceding claims, further configured to provide said other device with a web location to obtain a program to operate with said portable medical device.

9. The portable medical device (100) of any one of the preceding claims, containing a memory unit with a program that is downloadable to said other device to allow said other device to operate with said portable medical device.

10. The portable medical device (100) of any one of the preceding claims, configured to send, via said pairing, dynamic data of a medical procedure being carried out by said portable medical device.

11. The portable medical device (100) of any one of the preceding claims, configured to send, via said pairing, current state data of said portable medical device.

12. The portable medical device (100) of claim 11, wherein said current state data comprises at least one of temperature and readiness.

13. The portable medical device (100) of any one of the preceding claims, configured to send operational data at predetermined intervals as accumulated since a respective preceding interval, or to send all measurements as accumulated from a start of operation at each predetermined interval.

14. A system for carrying out a medical procedure, the system comprising:
the portable medical device (100) of any one of the preceding claims;
a remote server (102); and
another device, wherein said another device comprises an apparatus for connecting said portable medical device and a server, the apparatus comprising:
a communication unit;
a recognition unit configured to recognize identification information passively readable or held externally on said portable medical device or on the packaging (112) containing said device;
a pairing unit connected to said recognition unit to pair said apparatus with said portable medical device (100) when recognized following activation of the portable medical device (100) via automatic displacement of the battery isolator (204) from the portable medical device (100) upon opening of the packaging (112) containing the portable medical device (100), thereby opening a first channel (108) of communication with said portable medical device; wherein the communication unit is configured to open up a second channel (120) of communication with the server remotely over a network, thereby forming a communication route between said portable medical device and said server via said first and second communication channels (108, 120).

15. The system according to claim 14, wherein said recognition unit is configured to obtain data from said portable medical device.

16. The system according to claims 14 or 15, wherein said recognition unit comprises an RFID scanner or a barcode scanner.

17. The system according to claim 15, wherein said data obtained from said portable medical device via said first channel (108) and sent to said server via said second channel (120) is sufficient to allow said server (102) to identify said portable medical device (100).

18. The system according to claim 17, configured to obtain, via said first channel (108), dynamic data of medical process carried out by said portable medical device, and to pass on said dynamic data via said second channel (120) to said server (102).

19. The system according to claims 17 or 18, configured to receive data from said server (102) via said second channel (120) and to display said data to a user.

20. The system according to any one of claims 14 to 19, wherein the portable medical device (100) is an injector device.

21. The system according to any one of claims 14 to 20, configured to obtain an identification of said portable medical device (100) via said recognition unit, and to use said obtained identification to provide an identification prefix for communication data packets.

22. The system according to claim 21, wherein said communication data packets with said identification prefix are for said second communication channel (120).

23. The system according to claims 21 or 22, wherein said communication data packets with said identification prefix are for said first communication channel (108).

24. The system according to any one of claims 21, 22 and 23, wherein said identification prefix comprises data contained on said portable medical device (100).

25. The system according to any one of claims 21, 22 and 23, wherein said identification prefix comprises a derivation of data contained on said portable medical device (100).

26. The system according to claim 25, wherein said derivation comprises one-way hashing.

27. The system according to any one of claims 14 to 26, configured to accumulate operational data from said portable medical device (100) for sending to said server (102) at predetermined intervals, said operational data accumulation beginning at a start of operating said portable medical device (100).

28. The system according to claim 27, configured to send operational data, as accumulated from said start, at each of said predetermined intervals, or to send at each interval, measurements accumulated since a preceding interval.

29. The system according to any one claims 14 to 28, configured to receive a temperature measurement from said portable medical device (100) and to calculate a wait time for operating said portable medical device (100) based on said temperature measurement.

30. The system according to any one of claims 14 to 29, configured to receive from said portable medical device (100) at least one of an elapsed time, a load on a controlling motor, a substance volume, a pressure, a temperature, and a number of revolutions of said controlling motor and to display at least one of said elapsed time, a remaining time, a quantity of substance delivered and a quantity of substance remaining.

31. The system according to claim 30 configured to determine from said at least one of an elapsed time, a load on a controlling motor, a substance volume, a pressure, a temperature, and a number of revolutions of said controlling motor, that a procedure or dosage is incomplete and to indicate said incompleteness.

32. The system according to any one of claims 14 to 31, configured to determine that a procedure or dosage is complete and to display steps to be carried out following completion of said procedure or dosage.

33. The system according to any one of claims 14 to 32, configured to send said identification information to said server (102) to find further information at said server regarding a procedure or dosage indicated along with said portable medical device (100), said apparatus being further configured to retrieve and display said further information.

34. The system according to any one of claims 14 to 33, further configured to connect to said server to obtain a schedule for use of one or more of said portable medical devices and to provide schedule-related information including timed reminders.

35. A method of monitoring usage of a portable medical device (100), the method comprising providing a user with said system of any one of claims 14 to 34 and obtaining data regarding usage of said portable medical device (100) by said user and sending said data to said server (102).

36. The method according to claim 35, wherein the system is provided to each of a plurality of users, the plurality of users being volunteers in a clinical trial relating to the portable medical device (100), data regarding usage of said portable medical device (100) by respective volunteers is obtained and sent to said server (102).

37. The system according to claim 14, wherein said recognition unit is configured to obtain data from said portable medical device (100), said data comprising date information, thereby enabling said system to warn a user if said portable medical device (100) is out of date.

38. The system according to claim 14, wherein said portable medical device (100) is configured to obtain a temperature measurement or a temperature history therefrom to determine whether a substance contained therein is safe to use.

39. The system according to claim 14, wherein said portable medical device (100) is configured to provide over said first channel (108) at least one member of the group consisting of an elapsed time, a load on a controlling motor, a substance volume, a pressure, a temperature, and a number of revolutions of said controlling motor and said mobile device (100) is configured to display at least one member of the group consisting of said elapsed time or a remaining time or a quantity of substance delivered or a quantity of substance remaining.

40. The method according to claim 35, further comprising pairing the portable medical device (100) and connecting with the server (102) via a mobile telephony device, wherein the pairing and connecting includes:
reading identification information provided on said portable medical device (100) or on the packaging (112) containing said portable medical device (100), the identification information being provided for reading while said portable medical device is passive;
operating said portable medical device (100) and carrying out pairing with said portable medical device (100) based on said identification information following activation of the portable medical device (100) via automatic displacement of a battery isolator (204) from the portable medical device (100) upon opening of the packaging (112) containing the portable medical device (100);
connecting with said server (102) based on said identification information; and
providing guidance screens on said mobile telephony device based on data from said server (102) and said portable medical device (100) to guide the user to use said portable medical device (100).

## Patentansprüche

1. Tragbare medizinische Vorrichtung (100), aufweisend eine Einheit, um eine medizinische Prozedur durchzuführen, eine Überwachungseinheit, um die medizinische Prozedur zu überwachen, und eine Kommunikationseinheit, die konfiguriert ist, um mit einer anderen Vorrichtung zu koppeln, um Daten von der Überwachungseinheit zu senden, wobei die tragbare medizinische Vorrichtung mit passiver elektronisch lesbarer Information im Pack versehen ist, um es der anderen Vorrichtung zu ermöglichen, das Koppeln durchzuführen, wobei die tragbare medizinische Vorrichtung ferner einen Batterieisolator (204) aufweist, der eine Batterie von der Kommunikationseinheit trennt, wobei der Batterieisolator (204) konfiguriert ist, um auf ein Öffnen einer Verpackung, welche die tragbare medizinische Vorrichtung enthält, verlagert zu werden, wodurch die tragbare medizinische Vorrichtung auf das Öffnen der Verpackung aktiviert wird.

2. Tragbare medizinische Vorrichtung (100) gemäß Anspruch 1, wobei die medizinische Vorrichtung ein automatischer Injektor ist.

3. Tragbare medizinische Vorrichtung (100) gemäß Ansprüchen 1 oder 2, wobei die elektronisch lesbare Information ein Barcode (114), QR-Code oder ein RFID-Tag ist.

4. Tragbare medizinische Vorrichtung (100) gemäß Anspruch 3, wobei der Barcode (114), QR-Code oder RFID-Tag ein Teil der Verpackung ist, welche die tragbare medizinische Vorrichtung enthält.

5. Tragbare medizinische Vorrichtung (100) gemäß Anspruch 3, wobei der Barcode (114), QR-Code oder RFID an der tragbaren medizinischen Vorrichtung angebracht ist.

6. Tragbare medizinische Vorrichtung (100) gemäß irgendeinem der vorigen Ansprüche, wobei die Kommunikationseinheit konfiguriert ist, um das Koppeln durchzuführen basierend auf einem Empfangen von Koppeln-Information von der anderen Vorrichtung, welche auf der elektronisch lesbaren Information basiert.

7. Tragbare medizinische Vorrichtung (100) gemäß irgendeinem der vorigen Ansprüche, wobei die elektronisch lesbare Information Information aufweist, welche die tragbare medizinische Vorrichtung identifiziert, wodurch der anderen Vorrichtung ermöglicht wird, sich mit einem Server zu verbinden und die tragbare medizinische Vorrichtung zu identifizieren.

8. Tragbare medizinische Vorrichtung (100) gemäß irgendeinem der vorigen Ansprüche, die ferner konfiguriert ist, um die andere Vorrichtung mit einer Weblocation bereitzustellen, um ein Programm zu erlangen, um mit der tragbaren medizinischen Vorrichtung zu arbeiten.

9. Tragbare medizinische Vorrichtung (100) gemäß irgendeinem der vorigen Ansprüche, enthaltend eine Speichereinheit mit einem Programm, das in die andere Vorrichtung herunterladbar ist, um es der anderen Vorrichtung zu ermöglichen, mit der tragbaren medizinischen Vorrichtung zu arbeiten.

10. Tragbare medizinische Vorrichtung (100) gemäß irgendeinem der vorigen Ansprüche, die konfiguriert ist, um via das Koppeln dynamische Daten einer medizinischen Prozedur zu senden, die von der tragbaren medizinischen Vorrichtung durchgeführt wird.

11. Tragbare medizinische Vorrichtung (100) gemäß irgendeinem der vorigen Ansprüche, die konfiguriert ist, um via das Koppeln momentane Zustandsdaten der tragbaren medizinischen Vorrichtung zu senden.

12. Tragbare medizinische Vorrichtung (100) gemäß Anspruch 11, wobei die momentanen Zustandsdaten wenigstens eines von Temperatur oder Bereitschaft aufweisen.

13. Tragbare medizinische Vorrichtung (100) gemäß irgendeinem der vorigen Ansprüche, die konfiguriert ist, um Betriebsdaten in vorbestimmten Intervallen als akkumuliert seit einem jeweiligen vorigen Intervall zu senden, oder um alle Messungen als akkumuliert von einem Betriebsstart an bei jedem vorbestimmten Intervall zu senden.

14. System zum Durchführen einer medizinischen Prozedur, wobei das System aufweist:
die tragbare medizinische Vorrichtung (100) gemäß irgendeinem der vorigen Ansprüche,
einen Remote-Server (102) und
eine andere Vorrichtung, wobei die andere Vorrichtung eine Einrichtung zum Verbinden der tragbaren medizinischen Vorrichtung und eines Servers aufweist, wobei die Einrichtung aufweist:
eine Kommunikationseinheit,
eine Erkennungseinheit, die konfiguriert ist, um Identifikationsinformation zu erkennen, die passiv lesbar ist oder außen gehalten ist an der tragbaren medizinischen Vorrichtung oder an der Verpackung (112), welche die Vorrichtung enthält,
eine Kopplungseinheit, die mit der Erkennungseinheit verbunden ist, um die Einrichtung mit der tragbaren medizinischen Vorrichtung (100), wenn erkannt, zu koppeln folgend einer Aktivierung der tragbaren medizinischen Vorrichtung (100) via automatische Verlagerung des Batterieisolators (204) von der tragbaren medizinischen Vorrichtung (100) aus auf ein Öffnen der Verpackung (112), welche die tragbare medizinische Vorrichtung (100) enthält, wodurch ein erster Kommunikationskanal (108) mit der tragbaren medizinischen Vorrichtung geöffnet wird, wobei die Kommunikationseinheit konfiguriert ist, um einen zweiten Kommunikationskanal (120) mit dem entfernt liegenden Server zu öffnen über ein Netzwerk, wodurch ein Kommunikationsweg gebildet wird zwischen der tragbaren medizinischen Vorrichtung und dem Server via den ersten und den zweiten Kommunikationskanal (108, 120) .

15. System gemäß Anspruch 14, wobei die Erkennungseinheit konfiguriert ist, um Daten von der tragbaren medizinischen Vorrichtung zu erlangen.

16. System gemäß Ansprüchen 14 oder 15, wobei die Erkennungseinheit einen RFID-Scanner oder einen Barcode-Scanner aufweist.

17. System gemäß Anspruch 15, wobei die Daten, die von der tragbaren medizinischen Vorrichtung via den ersten Kanal (108) erlangt werden und via den zweiten Kanal (120) an den Server gesendet werden, ausreichen, um es dem Server (102) zu erlauben, die tragbare medizinische Vorrichtung (100) zu identifizieren.

18. System gemäß Anspruch 17, das konfiguriert ist, um via den ersten Kanal (108) dynamische Daten eines medizinischen Prozesses zu erlangen, der von der tragbaren medizinischen Vorrichtung durchgeführt wird, und die dynamischen Daten via den zweiten Kanal (120) an den Server (102) weiterzuleiten.

19. System gemäß Ansprüchen 17 oder 18, das konfiguriert ist, um Daten von dem Server (102) via den zweiten Kanal (120) zu empfangen und die Daten einem Benutzer anzuzeigen.

20. System gemäß irgendeinem der Ansprüche 14 bis 19, wobei die tragbare medizinische Vorrichtung (100) eine Injektionsvorrichtung ist.

21. System gemäß irgendeinem der Ansprüche 14 bis 20, die konfiguriert ist, um eine Identifikation der tragbaren medizinischen Vorrichtung (100) via die Erkennungseinheit zu erlangen und die erlangte Identifikation zu verwenden, um eine vorangestellte Identifikation für Kommunikationsdatenpakete bereitzustellen.

22. System gemäß Anspruch 21, wobei die Kommunikationsdatenpakete mit der vorangestellten Identifikation für den zweiten Kommunikationskanal (120) sind.

23. System gemäß Ansprüchen 21 oder 22, wobei die Kommunikationsdatenpakete mit der vorangestellten Identifikation für den ersten Kommunikationskanal (108) sind.

24. System gemäß irgendeinem der Ansprüche 21, 22 und 23, wobei die vorangestellte Identifikation Daten aufweist, die in der tragbaren medizinischen Vorrichtung (100) enthalten sind.

25. System gemäß irgendeinem der Ansprüche 21, 22 und 23, wobei die vorangestellte Identifikation eine Herleitung von Daten aufweist, die in der tragbaren medizinischen Vorrichtung (100) enthalten sind.

26. System gemäß Anspruch 25, wobei die Herleitung Eine-Richtung-Hashing aufweist.

27. System gemäß irgendeinem der Ansprüche 14 bis 26, das konfiguriert ist, um Betriebsdaten von der tragbaren medizinischen Vorrichtung (100) zu akkumulieren zum Senden an den Server (102) in vorbestimmten Intervallen, wobei die Betriebsdatenakkumulierung beim Betriebsstart der tragbaren medizinischen Vorrichtung (100) beginnt.

28. System gemäß Anspruch 27, das konfiguriert ist, um Betriebsdaten als akkumuliert vom Start an bei jedem der vorbestimmten Intervalle zu senden oder um bei jedem Intervall Messungen zu senden, die seit einem vorherigen Intervall akkumuliert sind.

29. System gemäß irgendeinem der Ansprüche 14 bis 28, das konfiguriert ist, um eine Temperaturmessung von der tragbaren medizinischen Vorrichtung (100) zu empfangen und eine Wartezeit zum Betreiben der tragbaren medizinischen Vorrichtung (100) zu berechnen basierend auf der Temperaturmessung.

30. System gemäß irgendeinem der Ansprüche 14 bis 29, das konfiguriert ist um von der tragbaren medizinischen Vorrichtung (100) wenigstens eines von einer verstrichenen Zeit, einer Last an einem Steuermotor, einem Substanzvolumen, einem Druck, einer Temperatur und einer Drehzahl des Steuermotors zu empfangen und um wenigstens eines von der verstrichenen Zeit, einer Menge an zugeführter Substanz und einer Menge an verbleibender Substanz anzuzeigen.

31. System gemäß Anspruch 30, das konfiguriert ist, um aus dem wenigstens einen von einer verstrichenen Zeit, einer Last an einem Steuermotor, einem Substanzvolumen, einem Druck, einer Temperatur und einer Drehzahl des Steuermotors zu ermitteln, dass eine Prozedur oder eine Dosierung unvollständig ist, und diese Unvollständigkeit anzuzeigen.

32. System gemäß irgendeinem der Ansprüche 14 bis 31, das konfiguriert ist, um zu ermitteln, dass eine Prozedur oder Dosierung vollständig ist, und um auszuführende Schritte anzuzeigen folgend der Vollendung der Prozedur oder Dosierung.

33. System gemäß irgendeinem der Ansprüche 14 bis 32, das konfiguriert ist, um die Identifikationsinformation an den Server (102) zu senden, um weitere Information beim Server zu finden bezüglich einer Prozedur oder Dosierung, die angegeben wird zusammen mit der tragbaren medizinischen Vorrichtung (100), wobei die Einrichtung ferner konfiguriert ist, um die weitere Information abzurufen und anzuzeigen.

34. System gemäß irgendeinem der Ansprüche 14 bis 33, das ferner konfiguriert ist, um Verbindung zum Server herzustellen, um einen Plan zur Verwendung einer oder mehrerer der tragbaren medizinischen Vorrichtungen zu erlangen und um planbezogene Information, umfassend zeitlich festgelegte Erinnerungen, bereitzustellen.

35. Verfahren zum Überwachen der Verwendung einer tragbaren medizinischen Vorrichtung (100), wobei das Verfahren aufweist Ausstatten eines Benutzers mit dem System gemäß irgendeinem der Ansprüche 14 bis 34 und Erlangen von Daten bezüglich der Verwendung der tragbaren medizinischen Vorrichtung (100) durch den Benutzer und Senden der Daten an den Server (102).

36. Verfahren gemäß Anspruch 35, wobei das System bereitgestellt ist an jeden einer Mehrzahl von Benutzern, wobei die Mehrzahl von Benutzern Freiwillige in einem klinischen Versuch sind, der sich auf die tragbare medizinische Vorrichtung (100) bezieht, wobei Daten bezüglich der Verwendung der tragbaren medizinischen Vorrichtung (100) durch jeweilige Freiwillige erlangt und an den Server (102) gesendet werden.

37. System gemäß Anspruch 14, wobei die Erkennungseinheit konfiguriert ist, um Daten von der tragbaren medizinischen Vorrichtung (100) zu erlangen, wobei die Daten Datumsinformation aufweisen, wodurch dem System ermöglicht wird, einen Benutzer zu warnen, falls die tragbare medizinische Vorrichtung (100) nicht mehr aktuell ist.

38. System gemäß Anspruch 14, wobei die tragbare medizinische Vorrichtung (100) konfiguriert ist, um eine Temperaturmessung oder eine Temperaturhistorie davon zu erlangen, um zu ermitteln, ob eine darin enthaltene Substanz sicher ist zum Verwenden.

39. System gemäß Anspruch 14, wobei die tragbare medizinische Vorrichtung (100) konfiguriert ist, um über den ersten Kanal (108) wenigstens ein Element der Gruppe bereitzustellen, die besteht aus einer verstrichenen Zeit, einer Last an einem Steuermotor, einem Substanzvolumen, einem Druck, einer Temperatur und einer Drehzahl des Steuermotors, und die bewegbare Vorrichtung (100) konfiguriert ist, um wenigstens ein Element aus der Gruppe anzuzeigen, die besteht aus der verstrichenen Zeit oder einer verbleibenden Zeit oder einer Menge an zugeführter Substanz oder einer Menge an verbleibender Substanz.

40. Verfahren gemäß Anspruch 35, ferner aufweisend Koppeln der tragbaren medizinischen Vorrichtung (100) und Verbinden mit dem Server (102) via eine mobile Telefonvorrichtung, wobei das Koppeln und Verbinden aufweist:
Lesen von Identifikationsinformation, die an der tragbaren medizinischen Vorrichtung (100) oder an der Verpackung (112), die die tragbare medizinische Vorrichtung (100) enthält, bereitgestellt ist, wobei die Identifikationsinformation zum Lesen bereitgestellt ist, während die tragbare medizinische Vorrichtung passiv ist,
Betreiben der tragbaren medizinischen Vorrichtung (100) und Durchführen eines Koppelns mit der tragbaren medizinischen Vorrichtung (100) basierend auf der Identifikationsinformation folgend einer Aktivierung der tragbaren medizinischen Vorrichtung (100) via automatische Verlagerung eines Batterieisolators (204) von der tragbaren medizinischen Vorrichtung (100) auf ein Öffnen der Verpackung (112), die die tragbare medizinische Vorrichtung (100) enthält,
Verbinden mit dem Server (102) basierend auf der Identifikationsinformation und
Bereitstellen von Führungsbildern auf der mobilen Telefonvorrichtung basierend auf Daten von dem Server (102) und der tragbaren medizinischen Vorrichtung (100), um den Benutzer anzuleiten, um die tragbare medizinische Vorrichtung (100) zu verwenden.

## Revendications

1. Dispositif médical portable (100) comprenant une unité pour effectuer une procédure médicale, une unité de surveillance pour surveiller ladite procédure médicale et une unité de communication configurée pour s'apparier avec un autre dispositif afin d'envoyer des données de ladite unité de surveillance, le dispositif médical portable étant conditionné avec des informations lisibles électroniquement passives pour permettre audit autre dispositif d'effectuer ledit appariement, le dispositif médical portable incluant en outre un isolateur de batterie (204) qui déconnecte une batterie depuis l'unité de communication, l'isolateur de batterie (204) étant configuré pour être déplacé lors de l'ouverture d'un conditionnement contenant le dispositif médical portable, en activant ainsi le dispositif médical portable lors de l'ouverture du conditionnement.

2. Dispositif médical portable (100) selon la revendication 1, dans lequel le dispositif médical est un injecteur automatique.

3. Dispositif médical portable (100) selon la revendication 1 ou 2, dans lequel lesdites informations lisibles électroniquement sont un code-barres (114), un code-barres bidimensionnel (QR code), ou une étiquette RFID.

4. Dispositif médical portable (100) selon la revendication 3, dans lequel le code-barres (114), le QR code ou l'étiquette RFID font partie du conditionnement contenant le dispositif médical portable.

5. Dispositif médical portable (100) selon la revendication 3, dans lequel le code-barres (114), le QR code ou la RFID est fixé(e) au dispositif médical portable.

6. Dispositif médical portable (100) selon l'une quelconque des revendications précédentes, dans lequel ladite unité de communication est configurée pour effectuer ledit appariement sur la base de la réception d'informations d'appariement en provenance dudit autre dispositif, sur la base desdites informations lisibles électroniquement.

7. Dispositif médical portable (100) selon l'une quelconque des revendications précédentes, dans lequel lesdites informations lisibles électroniquement incluent des informations identifiant ledit dispositif médical portable, en permettant ainsi audit autre dispositif de se connecter à un serveur et d'identifier ledit dispositif médical portable.

8. Dispositif médical portable (100) selon l'une quelconque des revendications précédentes, configuré en outre pour fournir audit autre dispositif un emplacement de bande pour obtenir un programme pour fonctionner avec ledit dispositif médical portable.

9. Dispositif médical portable (100) selon l'une quelconque des revendications précédentes, contenant une unité de mémoire avec un programme qui peut être téléchargé sur ledit autre dispositif afin de permettre audit autre dispositif de fonctionner avec ledit dispositif médical portable.

10. Dispositif médical portable (100) selon l'une quelconque des revendications précédentes, configuré pour envoyer, par l'intermédiaire dudit appariement, des données dynamiques d'une procédure médicale exécutée par ledit dispositif médical portable.

11. Dispositif médical portable (100) selon l'une quelconque des revendications précédentes, configuré pour envoyer, par l'intermédiaire dudit appariement, des données d'état actuel dudit dispositif médical portable.

12. Dispositif médical portable (100) selon la revendication 11, dans lequel lesdites données d'état actuel comprennent au moins une parmi la température et la disponibilité.

13. Dispositif médical portable (100) selon l'une quelconque des revendications précédentes, configuré pour envoyer des données opérationnelles à des intervalles prédéterminés telles qu'accumulées depuis un intervalle précédent respectif, ou pour envoyer toutes les mesures telles qu'accumulées à partir d'un début de fonctionnement à chaque intervalle prédéterminé.

14. Système de mise en œuvre d'une procédure médicale, le système comprenant :
le dispositif médical portable (100) selon l'une quelconque des revendications précédentes ;
un serveur distant (102) ; et
un autre dispositif, dans lequel ledit autre dispositif comprend un appareil pour connecter ledit dispositif médical portable et un serveur, l'appareil comprenant :
une unité de communication ;
une unité de reconnaissance configurée pour reconnaître des informations d'identification pouvant être lues passivement ou conservées à l'extérieur sur ledit dispositif médical portable ou sur le conditionnement (112) contenant ledit dispositif ;
une unité d'appariement connectée à ladite unité de reconnaissance pour apparier ledit appareil audit dispositif médical portable (100) lorsqu'il est reconnu suite à l'activation du dispositif médical portable (100) via un déplacement automatique de l'isolateur de batterie (204) à partir du dispositif médical portable (100) lors de l'ouverture du conditionnement (112) contenant le dispositif médical portable (100), en ouvrant ainsi un premier canal (108) de communication avec ledit dispositif médical portable ; dans lequel l'unité de communication est configurée pour ouvrir un second canal (120) de communication avec le serveur à distance sur un réseau, en formant ainsi un trajet de communication entre ledit dispositif médical portable et ledit serveur par l'intermédiaire desdits premier et second canaux de communication (108, 120).

15. Système selon la revendication 14, dans lequel ladite unité de reconnaissance est configurée pour obtenir des données à partir dudit dispositif médical portable.

16. Système selon la revendication 14 ou 15, dans lequel ladite unité de reconnaissance comprend un lecteur RFID ou un lecteur de code-barres.

17. Système selon la revendication 15, dans lequel lesdites données obtenues en provenance dudit dispositif médical portable par l'intermédiaire dudit premier canal (108) et envoyées audit serveur par l'intermédiaire dudit second canal (120) sont suffisantes pour permettre audit serveur (102) d'identifier ledit dispositif médical portable (100).

18. Système selon la revendication 17, configuré pour obtenir, par l'intermédiaire dudit premier canal (108), des données dynamiques d'un processus médical mis en œuvre par ledit dispositif médical portable, et pour transmettre lesdites données dynamiques par l'intermédiaire dudit second canal (120) audit serveur (102).

19. Système selon la revendication 17 ou 18, configuré pour recevoir des données en provenance dudit serveur (102) par l'intermédiaire dudit second canal (120), et pour afficher lesdites données à un utilisateur.

20. Système selon l'une quelconque des revendications 14 à 19, dans lequel le dispositif médical portable (100) est un dispositif injecteur.

21. Système selon l'une quelconque des revendications 14 à 20, configuré pour obtenir une identification dudit dispositif médical portable (100) par l'intermédiaire de ladite unité de reconnaissance, et pour utiliser ladite identification obtenue pour fournir un préfixe d'identification pour des paquets de données de communication.

22. Système selon la revendication 21, dans lequel lesdits paquets de données de communication avec ledit préfixe d'identification sont destinés audit second canal de communication (120).

23. Système selon les revendications 21 ou 22, dans lequel lesdits paquets de données de communication avec ledit préfixe d'identification sont pour ledit premier canal de communication (108).

24. Système selon l'une quelconque des revendications 21, 22 et 23, dans lequel ledit préfixe d'identification comprend des données contenues sur ledit dispositif médical portable (100).

25. Système selon l'une quelconque des revendications 21, 22 et 23, dans lequel ledit préfixe d'identification comprend une dérivation de données contenues sur ledit dispositif médical portable (100).

26. Système selon la revendication 25, dans lequel ladite dérivation comprend un hachage unidirectionnel.

27. Système selon l'une quelconque des revendications 14 à 26, configuré pour accumuler des données opérationnelles en provenance dudit dispositif médical portable (100) pour un envoi audit serveur (102) à des intervalles prédéterminés, ladite accumulation de données opérationnelles commençant au début du fonctionnement dudit dispositif médical portable (100).

28. Système selon la revendication 27, configuré pour envoyer des données opérationnelles, telles qu'accumulées depuis ledit début, à chacun desdits intervalles prédéterminés, ou pour envoyer à chaque intervalle des mesures accumulées depuis un intervalle précédent.

29. Système selon l'une quelconque des revendications 14 à 28, configuré pour recevoir une mesure de température en provenance dudit dispositif médical portable (100) et pour calculer un temps d'attente pour faire fonctionner ledit dispositif médical portable (100) sur la base de ladite mesure de température.

30. Système selon l'une quelconque des revendications 14 à 29, configuré pour recevoir en provenance dudit dispositif médical portable (100) au moins un parmi un temps écoulé, une charge sur un moteur de commande, un volume de substance, une pression, une température et un nombre de tours dudit moteur de commande et pour afficher au moins un dudit temps écoulé, d'un temps restant, d'une quantité de substance délivrée et d'une quantité de substance restante.

31. Système selon la revendication 30, configuré pour déterminer à partir dudit au moins parmi un temps écoulé, une charge sur un moteur de commande, un volume de substance, une pression, une température et un nombre de tours dudit moteur de commande, qu'une procédure ou un dosage est incomplet et pour indiquer ladite incomplétude.

32. Système selon l'une quelconque des revendications 14 à 31, configuré pour déterminer qu'une procédure ou un dosage est terminé(e) et pour afficher des étapes à mettre en œuvre après l'achèvement de ladite procédure ou dudit dosage.

33. Système selon l'une quelconque des revendications 14 à 32, configuré pour envoyer lesdites informations d'identification audit serveur (102) pour trouver des informations supplémentaires au niveau dudit serveur concernant une procédure ou un dosage indiqué(e) conjointement avec ledit dispositif médical portable (100), ledit appareil étant en outre configuré pour récupérer et afficher lesdites informations supplémentaires.

34. Système selon l'une quelconque des revendications 14 à 33, configuré en outre pour se connecter audit serveur pour obtenir un programme d'utilisation d'un ou plusieurs desdits dispositifs médicaux portables et pour fournir des informations relatives au programme incluant des rappels synchronisés.

35. Procédé de surveillance de l'utilisation d'un dispositif médical portable (100), le procédé comprenant la fourniture à un utilisateur dudit système selon l'une quelconque des revendications 14 à 34 et l'obtention de données concernant l'utilisation dudit dispositif médical portable (100) par ledit utilisateur, et l'envoi desdites données audit serveur (102).

36. Procédé selon la revendication 35, dans lequel le système est fourni à chacun d'une pluralité d'utilisateurs, la pluralité d'utilisateurs étant des volontaires dans un essai clinique relatif au dispositif médical portable (100), des données concernant l'utilisation dudit dispositif médical portable (100) par des volontaires respectifs sont obtenues et envoyées audit serveur (102).

37. Système selon la revendication 14, dans lequel ladite unité de reconnaissance est configurée pour obtenir des données en provenance dudit dispositif médical portable (100), lesdites données comprenant des informations de date, en permettant ainsi audit système d'avertir un utilisateur si ledit dispositif médical portable (100) est obsolète.

38. Système selon la revendication 14, dans lequel ledit dispositif médical portable (100) est configuré pour obtenir une mesure de température ou un historique de températures à partir de celui-ci afin de déterminer si une substance contenue dans celui-ci est d'une utilisation sûre.

39. Système selon la revendication 14, dans lequel ledit dispositif médical portable (100) est configuré pour fournir sur ledit premier canal (108) au moins un élément du groupe consistant en un temps écoulé, une charge sur un moteur de commande, un volume de substance, une pression, une température et un nombre de tours dudit moteur de commande, et ledit dispositif mobile (100) est configuré pour afficher au moins un élément du groupe consistant en ledit temps écoulé ou un temps restant ou une quantité de substance délivrée ou une quantité de substance restante.

40. Procédé selon la revendication 35, comprenant en outre l'appariement du dispositif médical portable (100) et la connexion avec le serveur (102) par l'intermédiaire d'un dispositif de téléphonie mobile, dans lequel l'appariement et la connexion comprennent les étapes consistant à :
lire des informations d'identification fournies sur ledit dispositif médical portable (100) ou sur le conditionnement (112) contenant ledit dispositif médical portable (100), les informations d'identification étant fournies pour lecture tandis que ledit dispositif médical portable est passif ;
faire fonctionner ledit dispositif médical portable (100) et mettre en œuvre un appariement avec ledit dispositif médical portable (100) sur la base desdites informations d'identification suite à une activation du dispositif médical portable (100) via un déplacement automatique d'un isolateur de batterie (204) à partir du dispositif médical portable (100) lors de l'ouverture du conditionnement (112) contenant le dispositif médical portable (100) ;
se connecter audit serveur (102) sur la base desdites informations d'identification ; et
fournir des écrans de guidage sur ledit dispositif de téléphonie mobile sur la base de données en provenance dudit serveur (102) et dudit dispositif médical portable (100) afin de guider l'utilisateur pour utiliser ledit dispositif médical portable (100).
